# NEW EUROPEAN PATENT SPECIFICATION

(11) **EP 2 100 590 B2**
(45) Date of publication and mention of the opposition decision: **04.10.2017**
(45) Mention of the grant of the patent: 01.01.2014
(21) Application number: 07831834.2
(22) Date of filing: 14.11.2007
(51) Int. Cl.: A61K 8/73, A61Q 11/00

(54) **ORAL COMPOSITION CONTAINING CRYSTALLINE CELLULOSE SURFACE-TREATED WITH WATER-SOLUBLE SUBSTANCE**
ORALE ZUSAMMENSETZUNG MIT KRISTALLINER CELLULOSE, DIE MIT EINER WASSERLÖSLICHEN SUBSTANZ OBERFLÄCHENBEHANDELT IST
COMPOSITION ORALE CONTENANT DE LA CELLULOSE CRISTALLINE TRAITÉE EN SURFACE AVEC UNE SUBSTANCE SOLUBLE DANS L'EAU

(30) Priority: 14.11.2006 JP 2006308458
(43) Date of publication of application: 16.09.2009
(73) Proprietor: Sunstar Inc., Takatsuki-shi Osaka 569-1195 (JP)
(72) Inventor: NAKAO, Akira, Takatsuki-shi Osaka 569-1044 (JP); HASEGAWA, Noritaka, Takatsuki-shi Osaka 569-1044 (JP)
(74) Representative: Stoner, Gerard Patrick
(86) International application number: PCT/JP2007/072104
(87) International publication number: WO 2008/059881

(56) References cited:
- EP-A1- 1 839 649
- WO-A1-98/30209
- WO-A2-2004/071321
- WO-A2-2007/129329
- JP-A- 2004 339 180
- JP-A- 2005 200 327
- US-A- 4 216 242
- US-A- 5 601 803
- US-A1- 2005 244 346

## Description

### BACKGROUND OF THE INVENTION

### Field of the Invention

The present invention relates to oral hygiene compositions. More particularly, the present invention relates to oral hygiene compositions, particularly dentifrice compositions, which contain microcrystalline cellulose which is surface-treated with a water-soluble substance and display excellent shape retention and dispersibility in the oral cavity.

### Description of the Related Art

It is required for a dentifrice composition to have not only utility as an auxiliary cleaning agent for a toothbrush, but also utility as a preparation for oral cavity health, to have positive effects of prevention/treatment of diseases in the oral cavity, prevention of malodor of the oral cavity and whitening exerted by blending ingredients having various actions. However, even if such ingredients having various actions are blended, the utilities are not sufficiently exerted unless the ingredients are immediately dispersed throughout the oral cavity when the dentifrice composition is applied to the oral cavity, and thus the object cannot be achieved.

In general, the dosage form of a composition suited to disperse such ingredients throughout the oral cavity is a liquid oral hygiene composition. However, a liquid oral hygiene composition entails the problem that it is difficult to physically remove dental plaques and stains since an abrasive is not usually blended.

In contrast, a toothpaste can be easily blended with an abrasive to form a formulation suited to physically remove dental plaques and stains when used together with a toothbrush (JP-A No. 2004-10576). However, since a large amount of a thickener is blended so as to prevent solid-liquid separation of the abrasive, and the resultant toothpaste has comparatively high viscosity, a level of brushing time is required so as to disperse the blended ingredients throughout the oral cavity.

Therefore, there is a problem that the expected action of the blended ingredients is not sufficiently exerted when the brushing time by users of the toothpaste is short, or the brushing technique is poor. Since most dentifrice ingredients are washed away after cleaning, it is desirable that pharmacologically active ingredients acting on biotissues are immediately dispersed throughout the oral cavity when the toothpaste is used so as to make the contact time with the target tissues as long as possible. In order to stably place a dentifrice on a toothbrush and use it in a preferred manner, it is required to have high shape retention and low stringiness, which conflict with high dispersibility.

Therefore, there is a need for a dentifrice composition which is blended with an abrasive, particularly a dentifrice composition in which various ingredients are immediately dispersed throughout the oral cavity after the dentifrice composition is applied while maintaining physical properties such as high shape retention and low stringiness, allowing for sufficient realization of their action.

It is usually considered that the amount of a thickener blended is decreased to form a composition having low viscosity so as to prepare a composition having high dispersibility. However, a composition having low viscosity has a problem that ingredients such as an abrasive become sedimented thereby causing deterioration of stability of the composition and sinking between bristles when placed on a toothbrush, and also spinning occurs and thus a feeling for use deteriorates.

Our US2005/02443646 A (see also JP 2002/179541 A and EP 1532971 A) discloses oral compositions e.g. in the form of toothpaste, dentifrice, gel, spray or foam and comprising crystalline cellulose e.g. microcrystalline cellulose. Other optional components include thickening agents which may be cellulose derivatives or synthetic polymers.

WO2004/071321 describes toothpastes containing microcrystalline cellulose, a binder, and surface active agent selected from hydrocolloids such as carboxymethyl cellulose, hydroxyethyl cellulose, xanthan gum, guar gum and maltodextrin.

JP-A-2004/339180 describes oral compositions containing microcrystalline cellulose in aqueous dispersion, viscosity of 50 to 1000 mPa·s and various optional blended additives, including water-soluble polymers such as sodium carboxymethyl cellulose as an option, as in the above-cited JP-A-2004/010576.

### SUMMARY OF THE INVENTION

An object of the present invention is to provide an oral hygiene composition which has high shape retention and low stringiness, and also enables blended ingredients to disperse immediately throughout the oral cavity.

In the light of the above-stated circumstances, the present inventors have intensively studied and found that such an object can be achieved by blending microcrystalline cellulose which is surface-treated with a water-soluble substance, and thus the present invention has been completed.

That is, the present invention provides:
[1] An oral hygiene composition as defined in claim 1, to be used by ejecting onto a toothbrush from a pump container, the composition comprising surface-treated microcrystalline cellulose having a coating of water-soluble polymer which is sodium carboxymethyl cellulose, contained at a weight ratio of microcrystalline cellulose to said sodium carboxymethyl cellulose in the range from 4:1 to 15:1;
   in which the amount of the surface-treated microcrystalline cellulose blended in the oral hygiene composition is within a range from 3 to 7% by weight based on the total amount of the composition; and
   the composition further contains a pharmacologically active ingredient which is one or more selected from the group consisting of cetylpyridinium chloride, benzethonium chloride, distearyldimonium chloride, stearalkonium chloride, steartrimonium chloride, steartrimonium chloride, laurylpyridinium chloride, chlorhexidine dihydrochloride, chlorhexidine acetate, chlorhexidine digluconate, alexidine hydrochloride, alexidine acetate, alexidine gluconate, triclosan, O-cymen-5-ol, enzymes, zinc compounds, vitamins and derivatives thereof, amino acids, types of collagen, 6-aminocaproic acid, allantoin and derivatives thereof, dihydrocholesterol, glycyrrhizates, glycyrrhetinic acid, glycerophosphates and chlorophyll.
[2] The oral hygiene composition according to aforementioned [1], wherein the pharmacologically active ingredient is one or more selected from the group consisting of cetylpyridinium chloride, triclosan, vitamin E and a derivative thereof, tranexamic acid and glycyrrhizate;
[3] The oral hygiene composition according to aforementioned [1] or [2], which further contains an abrasive;
[4] The oral hygiene composition according to any one of aforementioned [1] to [3], which further contains a surfactant;
[5] The oral hygiene composition according to any one of aforementioned [1] to [4], wherein viscosity at 30°C of the composition is from 5 to 20 Pa·s.

The invention can impart high shape retention and low stringiness, and also enable various blended ingredients to immediately disperse throughout the oral cavity when it is applied to the oral cavity, without causing sinking between tufts when placed on a toothbrush, thus providing an oral hygiene composition which can be used with an excellent feeling for use, and also can sufficiently exert action of various ingredients, within a short time.

The invention enables a pharmacologically active ingredient and an abrasive to easily disperse throughout the oral cavity within a short time because of lower stringiness, thus making it possible to provide an oral hygiene composition which can sufficiently exert actions of active ingredients throughout the oral cavity, and also can easily remove dental plaques and stains adhered to the tooth surfaces within a short time and can also impart an excellent feeling for use.

The features according to aforementioned [1] and [2] enable the various pharmacologically active ingredients to easily disperse throughout the oral cavity, thus making it possible to provide an oral hygiene composition which can effectively wash the oral cavity and can prevent or treat diseases in the oral cavity more effectively.

The features according to aforementioned [3] and [4] can provide an oral hygiene composition which can be formulated into various forms, and also can easily remove dental plaques and stains adhered to the tooth surface within a short time and has excellent storage stability.

The feature according to aforementioned [5] imparts viscosity in the given range making it possible to provide an oral hygiene composition in a new dosage form, which enables various blended ingredients to easily disperse throughout the oral cavity, and is also excellent in a feeling for use.

The present application can provide an oral hygiene composition which can always eject a given amount of the composition without varying the amount depending on individuals, and also can exert the expected effects of the composition since the composition is applied by ejecting onto a toothbrush from a pump container.

### DETAILED DESCRIPTION OF THE INVENTION

Microcrystalline cellulose which is surface-treated with a water-soluble substance (hereinafter may be referred to as "surface-treated microcrystalline cellulose") to be used in the oral hygiene composition of the present invention is originally treated by coating the surface of microcrystalline cellulose having low affinity with water with a water-soluble substance, so that the microcrystalline cellulose is dispersed in a colloidal form in water by stirring in water as a result of swelling and dissolution of the water-soluble substance, although microcrystalline cellulose by itself is precipitated in water. Surface-treated microcrystalline cellulose can be produced, for example, by hydrolysing a high-purity pulp to extract crystalline regions and wet-mixing with the water-soluble substance, followed by drying, and usually has a granular form.

Microcrystalline cellulose of surface-treated microcrystalline cellulose is produced by extracting crystalline regions of cellulose which is obtained by acid hydrolysis or alkali hydrolysis of pulp fibers of plants, followed by purification. When surface-treated microcrystalline cellulose is dispersed in water, the average particle diameter thereof is within a range from 1 to 50 µm, preferably from 1 to 12 µm, and more preferably from 3 to 10 µm.

The water-soluble substance to be used in the surface treatment of the microcrystalline cellulose is sodium carboxymethyl cellulose, used alone or in combination of two or more. Other water-soluble substances are polymers and sugar alcohols, for example, cellulose derivatives such as hydroxyethylcellulose; microbiological polymers such as xanthan gum, natural polymers such as Sterculia urens gum, dextrin and carrageenan, and sugar alcohols such as sorbitol, xylitol, erythritol and isomalt.

The ratio of microcrystalline cellulose and the water-soluble substance (sodium carboxymethyl cellulose) constituting surface-treated microcrystalline cellulose, microcrystalline cellulose:water-soluble substance (total amount of one or more), is within a range from 4:1 to 15:1 (weight ratio), preferably from 5:1 to 15:1. When the ratio of microcrystalline cellulose is more than the above range, dispersion of microcrystalline cellulose becomes insufficient when contacted with water, resulting in low shape retention. In contrast, when the ratio of microcrystalline cellulose is less than the above range, viscosity increases and it becomes impossible to provide an oral hygiene composition having high dispersibility.

As surface-treated microcrystalline cellulose, for example, those with various grades such as Ceolus^{®} RC series (average particle diameter: 8 to 10 µm) and SC series (average particle diameter: 7 to 9 µm) commercially available from Asahi Kasei Chemicals Corporation, etc. can be used. Also, Avicel^{®} RC series, CL series and SG series, commercially available from FMC Corporation and Neocel^{®} commercially available from Mingtai Chemical Co., Ltd. can be used.

The amount of surface-treated microcrystalline cellulose to be blended in the oral hygiene composition of the present invention is within a range from 3 to 7% by weight, based on the total amount of the composition. When the amount of surface-treated microcrystalline cellulose is less, since it becomes impossible to impart favorable shape retention to the oral hygiene composition, it becomes difficult to determine an appropriate use amount as a result of sinking when it is placed on a toothbrush, and also dispersion in the oral cavity is delayed. In contrast, when the amount of surface-treated microcrystalline cellulose is more, viscosity of the oral hygiene composition increases and dispersibility in the oral cavity deteriorates.

It is also possible to use two or more kinds of surface-treated microcrystalline cellulose in combination, each having a different raw material ingredient or composition.

Viscosity at 20°C of a 1 wt% aqueous solution of the surface-treated microcrystalline cellulose is usually within a range from 5 to 100 mPa·s when measured at a rotating speed of 20 rpm/min. using a Brookfield rotary viscometer and a rotor No. 1.

Usually, the average particle diameter after dispersion of surface-treated microcrystalline cellulose is preferably within a range from 3 to 10 µm when measured by a laser diffraction/scattering type particle size distribution analyzer.

Examples of ingredients which are used in combination with the aforementioned surface-treated microcrystalline cellulose in the oral hygiene composition of the present invention, and are favorably dispersed throughout the oral cavity, include (in addition to the pharmacologically active ingredients) abrasives, surfactants, flavors, sweeteners, etc. When these ingredients can be favorably dispersed throughout the oral cavity, it is possible to exert the action of these ingredients at more sites for a longer time.

Among these ingredients, the pharmacologically active ingredient includes one or more of those specified in claim 1. Other (optional) pharmacologically active ingredients include fluoride compounds such as sodium monofluorophosphate, potassium monofluorophosphate, sodium fluoride, potassium fluoride, ammonium fluoride, stannous fluoride, etc.; potassium nitrate and sodium chloride. These pharmacologically active ingredients can be used alone or in combination of two or more, and the amount thereof is an amount which is usually considered that each pharmacologically active ingredient exerts the objective pharmacological effect.

Examples of the abrasive include abrasive silicas such as abrasive precipitated silica, abrasive gel silica, etc., dicalcium phosphate dihydrate and anhydrous, calcium phosphate, tricalcium phosphate, calcium carbonate, calcium pyrophosphate, calcium silicate, aluminum hydroxide, alumina, aluminum silicate, pumice, insoluble sodium metaphosphate, trimagnesium phosphate, magnesium carbonate, calcium sulfate, poly(methyl methacrylate), bentonite, zirconium silicate, hydroxyapatite, synthetic resins, etc. Among these abrasives, abrasive silica, dicalcium phosphate dihydrate and anhydrous, calcium carbonate, calcium pyrophosphate, aluminum hydroxide, alumina and hydroxyapatite are preferred, abrasive silica, dicalcium phosphate dihydrate and anhydrous, and alumina are more preferred, and abrasive silica is most preferred. These abrasives can be used alone or in combination of two or more, and the amount thereof is usually within a range from 4 to 40% by weight based on the total amount of the oral hygiene composition.

Examples of the surfactant include higher alkyl sulfate salts having alkyl groups including 8 to 18 carbon atoms, such as sodium lauryl sulfate, sodium myristyl sulfate, etc.; and anionic surfactants such as N-long chain acyl amino acid salts, α-olefin sulfonates, higher fatty acid sodium monoglyceride monosulfates, N-methyl-N-palmitoyl taurides, sodium N-acylsarcosinates, N-acyl glutamates, sodium N-methyl-N-acyl taurates, sodium N-methyl-N-acyl aminopropionates, etc. In addition to the above anionic surfactants, surfactants generally used in the oral hygiene composition can be used and examples of such the surfactants include nonionic surfactants such as polyoxyethylene sorbitan esters of fatty acid (for example, Polysorbate 20, etc.), polyoxyethylene hydrogenated castor oil, Lauramide MEA, Myristamide MEA, polyoxyethylene higher alcohol ether, Poloxamer, alkylglycosides (for example, alkyl chain: about C8 to C16), polyglycerol esters of fatty acid (for example, alkyl chain of the fatty acid moiety: about C8 to C16), sucrose esters of fatty acid (for example, alkyl chain of the fatty acid moiety: about C8 to C16), etc.; amphoteric surfactants such as N-alkyl diaminoethylglycines, alkyl betaines, fatty acid amidopropyl betaines (for example, alkyl chain of the fatty acid moiety: about C8 to C16), alkyl sulfobetaines, alkyl betaines, imidazolinium betaines, etc.; and cationic surfactants such as Behentrimonium chloride, Behentrimonium bromide, alkyldimethylammonium chloride, etc. These surfactants can be used alone or in combination of two or more, and the amount thereof is usually within a range from 0.001 to 5% by weight based on the total amount of the oral hygiene composition.

Examples of the flavor include menthol, carvone, anethole, vanillin, benzyl succinate, eugenol, methyl salicylate, limonene, ocimene, n-decyl alcohol, citronellol, α-terpineol, methyl acetate, citronellyl acetate, methyl eugenol, cineol, linalool, ethyllinalool, thymol, thyme, nutmeg, spearmint oil, peppermint oil, star anise oil, fennel oil, lemon oil, orange oil, sage oil, rosemary oil, cinnamon oil, pimento oil, diatom oil, perilla oil, wintergreen oil, clove oil, eucalyptus oil, basil oil, tea tree oil, davana oil, etc. These flavors can be used alone or in combination of two or more, and the amount thereof is usually within a range from 0.01 to 5% by weight based on the total amount of the oral hygiene composition.

Examples of the sweetener include saccharin, sodium saccharin, stevia extract, stevioside, acesulfame K, glycyrrhizin, glycyrrhizates, perillartine, thaumatin, aspartyl phenylalanine methyl ester, xylitol, paratinose, isomalt, erythritol, maltitol, etc. These sweeteners can be used alone or in combination of two or more, and the amount thereof is usually within a range from 0.001 to 50% by weight based on the total amount of the oral hygiene composition.

The oral hygiene composition of the present invention can appropriately contain, in addition to the above ingredients to be blended for the purpose of favorably dispersing throughout the oral cavity, ingredients which are usually blended with the oral hygiene composition, for example, binders, humectants, pH adjusting agents, preservatives, colorants or pigments and water as long as the effects of the present invention are not deteriorated.

Examples of the binder include cellulose derivatives such as hydroxyethylcellulose, hydroxypropylcellulose, a carboxymethylethyl cellulose salt, etc.; microbiological polymers, such as xanthan gum, etc.; natural polymers or natural rubbers, such as Sterculia urens gum, dextrin, carrageenan, tragacanth gum, arabic gum, gellan gum, etc.; synthetic polymers such as polyvinyl alcohol, polyvinyl pyrrolidone, etc.; inorganic binders such as thickening silica, magnesium aluminum silicate, etc.; and cationic binders such as Polyquaternium-10, etc. These binders can be used alone or in combination of two or more, and the amount thereof is usually within a range from 0.001 to 10% by weight based on the total amount of the oral hygiene composition.

Examples of the humectant include glycerin, sorbitol, Glycol, propylene glycol, butylene glycol, polyethylene glycol, polypropylene glycol, xylitol, maltitol, lactitol, isomalt, etc. These humectants can be used alone or in combination of two or more, and the amount thereof is usually within a range from 0.01 to 90% by weight based on the total amount of the oral hygiene composition.

Examples of the pH adjusting agent include citric acid, phosphoric acid, malic acid, pyrophosphoric acid, lactic acid, tartaric acid, glycerophosphoric acid, acetic acid, nitric acid, silicic acid, gluconic acid, maleic acid, aspartic acid, succinic acid, glucuronic acid, fumaric acid, adipic acid, glutamic acid, or chemically-acceptable salts thereof, such as sodium and potassium salts, hydrochloric acid, sodium hydroxide, potassium hydroxide, sodium silicate, etc. These pH adjusting agents can be used alone or in combination of two or more so as to adjust the pH of the oral hygiene composition within a range from 5 to 9. The amount thereof is usually within a range from 0.01 to 5% by weight based on the total amount of the oral hygiene composition.

Examples of the preservative include benzoic acid salts such as sodium benzoate, etc.; and parabens such as methylparaben, butylparaben, etc. These preservatives can be used alone or in combination of two or more, and the amount thereof is usually within a range from 0.01 to 3% by weight based on the total amount of the oral hygiene composition.

Examples of the colorant or pigment include pigments such as titanium dioxide, Ultramarines, Iron blue, etc.; and authorized dyes such as CI 42090, CI 45430, CI 45410, CI 15850, CI 73360, CI 19140, Ci47005, etc. These colorants or pigments can be used alone or in combination of two or more.

Viscosity at 30°C of the oral hygiene composition of the present invention is usually within a range from 5 to 50 Pa·s, preferably from 5 to 20 Pa·s, and more preferably from 7 to 15 Pa·s. As long as the effects of the present invention are not deteriorated, the oral hygiene composition can be formulated into forms such as dentifrice including toothpaste, gel, paste for oral cavity, cream, etc., and can be filled in pump containers.

The oral hygiene composition of the present invention (particularly toothpaste composition) is for use in a pump container, and viscosity at 30°C is preferably adjusted within a range from 5 to 20 Pa·s. The dentifrice composition having viscosity within the above range belongs to a dentifrice composition having low viscosity. Although a conventional toothpaste composition has viscosity of more than 20 Pa·s, dispersibility of the active ingredients can be improved by adjusting viscosity within a range of 20 Pa·s or less in the present invention. In an existing dentifrice composition, desirable shape retention cannot be imparted to the dentifrice composition only by decreasing viscosity. However, according to the present invention, dispersibility is improved by blending surface-treated microcrystalline cellulose while accomplishing shape retention in a low viscosity region. However, when viscosity is less than 5 Pa·s, sufficient shape retention cannot be obtained.

As the container for toothpaste, tube containers such as laminate tubes and aluminium tubes are commonly used. These tube containers have a mechanism in which contents are ejected through a container outlet when a compressive force is applied to the body by users using their hands. However, the used amount varies with each user, or varies with the time even when it is used by the same user. In a dentifrice, particularly a dentifrice containing a pharmacologically active ingredient, it is important that the effective amount of the dentifrice is applied to the oral cavity. However, there is a problem that it is difficult to uniformly adjust the amount of the dentifrice per application in the tube container and therefore problems arise with respect to an insufficient amount being used for brushing when there is a small amount of dentifrice and conversely economy problems caused by use of an amount of dentifrice above the effective amount when there is a large amount of the dentifrice.

A pump container has an advantage that a uniform amount of the dentifrice is ejected by a single extrusion operation and the above problem such as non-uniform amount per one time can be solved. However, even if the toothpaste is contained in a general-purpose pump container such as a shampoo container, an inconvenience arises in that the pumping portion does not smoothly return the toothpaste, or it becomes impossible to eject it using a pump. Furthermore there is the a problem that a large amount of the toothpaste remains, resulting in poor economy since the toothpaste has adhesion and viscosity and also has characteristics that it contains a large amount of a powder. Therefore, it is not easy to use a pump container as the container for toothpaste compositions at present.

However, the dentifrice compositions of the present invention can be favorably stored in or ejected from a pump container. A dentifrice composition of the present invention has low viscosity and an appropriate thixotropic nature and is therefore easily ejected by pumping, and also causes neither collapse of the shape nor sag from a toothbrush when it is placed on the toothbrush, and causes less adhesion of a powder due to drying in a pump delivery portion. Also in the pump container, the dentifrice composition is favorably accumulated to a pump suction port and is easily ejected from a pump. In pump containers, there often arise an inconvenience that a suction pressure enables a dentifrice as contents to cause reverse suction of a piston in the pump and the pumping portion does not smoothly return, or an inconvenience that the contents are partially solidified in the container or the pump mechanism and thus it becomes impossible to eject by means of pumping. However, a dentifrice composition of the present invention has low viscosity and favorable slipperiness and is therefore free from these inconveniences.

Although there is no particular limitation on the kind of the pump container, the pump container preferably has a structure comprising at least two layers of an inflexible outer layer and a flexible inner layer in which the inner layer is compressed as contents are ejected, thus preventing air from entering into the content sealed portion (delaminated structure). With such a structure, the contents can be easily ejected by pressing using hands and fingers and a pharmacologically active ingredient can be stably maintained even after sealing since it scarcely comes into contact with the air.

Next, the present invention will be described in more detail by way of examples, which are for illustrative purpose only and not to be construed restrictively.

### Examples

### Experimental Example 1: Shape Retention and Stringiness of Composition

### Preparation of Oral hygiene composition

An oral hygiene composition containing surface-treated microcrystalline cellulose suitable to be used in the present invention (Example 1) and an oral hygiene composition (same raw materials and same blending composition) using microcrystalline cellulose and a water-soluble substance alone (Comparative Example 1) were prepared according to formulations shown in the following tables.

A laminate tube was filled with each prepared oral hygiene composition and then shape retention and stringiness were tested.

**Table 1**

| Ingredients | Example 1 (Reference) | Comparative Example 1 |
|---|---|---|
| Microcrystalline cellulose (sodium carboxymethyl cellulose surface treatment, microcrystalline cellulose:sodium carboxymethyl cellulose = 89:11 (weight ratio)) | 5.6 | - |
| Microcrystalline cellulose | - | 5 |
| Sodium carboxymethyl cellulose | - | 0.6 |
| Dicalcium phosphate dihydrate | 30 | 30 |
| Glycerin | 20 | 20 |
| Sodium lauryl sulfate | 1.5 | 1.5 |
| Flavor | 1 | 1 |
| Sodium saccharin | 0.3 | 0.3 |
| Purified water | balance | balance |
| Total | 100 | 100 |
| Shape retention (mm) (after 1 minute) | 21 | 3 |
| Stringiness (mm) | 3.1 | 9.5 |
| Viscosity (Pa·s) | 36 | 6.4 |

### Evaluation of Shape Retention

A cap having an election port diameter of 3 mm was attached to a port of a laminate tube and an oral hygiene composition was ejected so as to be laid on a wire of a shape retention measuring device (rectangular metal rack in which wires are arranged in the form of a bar at different intervals). After a lapse of a predetermined time, a maximum distance between wires, at which the oral hygiene composition was not broken and dropped, was measured. With respect to each formulation, the measurement was repeated 5 times and an average distance between wires was calculated.

### Evaluation of Stringiness

After placing 50 mL of a prepared oral hygiene composition in a beaker, a stainless steel ball having a diameter of 16 mm was pressed into the position located 8 mm from the top face of the composition. The stainless steel ball was raised at a rate of 1,000 mm/min. and the oral hygiene composition adhered to the stainless steel ball was allowed to cause stringing, and then a distance between the position of the stainless steel ball and the position of the top face of the composition when the thread was broken was measured. These results are shown in Table 1.

As is apparent from the results of Table 1, the oral hygiene composition of Example 1 shows higher shape retention and lower stringiness than the oral hygiene composition of Comparative Example 1.

### Experimental Example 2: Dispersibility of Ingredients

### Preparation of Oral hygiene composition

An oral hygiene composition using a microcrystalline cellulose surface-treated with a water-soluble substance to be used in the present invention (Example 2) and an oral hygiene composition (same raw materials) using a microcrystalline cellulose and a water-soluble substance alone so as to obtain same shape retention (Comparative Example 1) were prepared according to formulations shown in the following Table 2.

**Table 2**

| Ingredients | Example 2 | Comparative Example 2 |
|---|---|---|
| Microcrystalline cellulose (sodium carboxymethyl cellulose surface treatment, microcrystalline cellulose:sodium carboxymethyl cellulose = 89:11 (weight ratio)) | 4 | - |
| Microcrystalline cellulose | - | 3.56 |
| Sodium carboxymethyl cellulose | - | 2.04 |
| Silica | 10 | 10 |
| Glycerin | 30 | 30 |
| Sorbitol | 20 | 20 |
| Sodium lauryl sulfate | 1 | 1 |
| Cetylpyridinium chloride | 0.1 | 0.1 |
| Tranexamic acid | 0.1 | 0.1 |
| Dipotassium glycyrrhizate | 0.1 | 0.1 |
| Vitamin E (tocopheryl acetate) | 0.1 | 0.1 |
| Triclosan | 0.1 | 0.1 |
| Sodium fluoride | 0.2 | 0.2 |
| Flavor | 1 | 1 |
| Sodium saccharin | 0.2 | 0.2 |
| Purified water | balance | balance |
| Total | 100 | 100 |
| Shape retention (after 20 seconds) | 12 | 12 |
| Stringiness | 3.1 | 48.0 |
| Viscosity (Pa·s) | 12 | 30 |

As is apparent from the results shown in Table 2, the oral hygiene composition of Example 2 has remarkably low stringiness and lowered viscosity when compared with the oral hygiene composition of Comparative Example 2, although these are oral hygiene compositions prepared so as to have equivalent shape retention.

### Dispersibility Test 1

Each of 4 g of oral hygiene compositions of Example 2 and Comparative Example 2 prepared was placed in a test container and 12 mL of an artificial saliva (50 mM KCl, 1 mM KH₂PO₄, 1 mM CaCl₂, 0.1 mM MgCl₂) was added, followed by shaking in a shaker (250 times/min.) for 5 seconds to 5 minutes. The solution obtained after shaking was filtered and methanol was added to make 25 mL. The solution was filtered again using a membrane filter and the filtrate was taken as a sample solution. Separately, to 2 mL of a 4-fold slurry solution of an oral hygiene composition, methanol was added to make 25 mL, and the filtrate obtained by filtration with a membrane filter was taken as a standard solution. The amount of cetylpyridinium chloride of each solution was determined by HPLC and a filtered elution rate (%) = (amount of cetylpyridinium chloride in sample solution)/(amount of cetylpyridinium chloride in standard solution) was calculated. The results are shown in Table 3.

**Table 3**

| Shaking time | Elution rate (%) | |
|---|---|---|
| | Example 2 | Comparative Example 2 |
| 5 seconds | 1 | 1 |
| 15 seconds | 5 | 1 |
| 30 seconds | 22 | 2 |
| 60 seconds | 20 | 3 |
| 180 seconds | 85 | 8 |
| 300 seconds | 100 | 11 |

As is apparent from the results shown in Table 3, the oral hygiene composition of Example 2 showed enhanced dispersibility from 5 seconds after shaking and is completely dispersed after 5 minutes. In contrast, the oral hygiene composition of Comparative Example 2 showed low dispersibility. After 5 minutes when the oral hygiene composition of Example 2 was completely dispersed, only 11% of the oral hygiene composition was dispersed.

### Preparation of Oral hygiene composition (Reference)

Oral hygiene compositions using microcrystalline cellulose surface-treated with a water-soluble substance outside the present invention (Reference Examples 3 and 4) and an oral hygiene composition (same materials) using microcrystalline cellulose and a water-soluble substance alone so as to obtain same shape retention (Comparative Example 3) were prepared according to formulations shown in the following Table 4. Then, shape retention, stringiness and viscosity thereof were measured by the methods described above.

**Table 4**

| Ingredients | Example 3 (Reference) | Example 4 (Reference) | Comparative Example 3 |
|---|---|---|---|
| Microcrystalline cellulose (xanthan gum and dextrin surface treatment, microcrystalline cellulose:xanthan gum:dextrin = 75:5:20 (weight ratio)) | 5 | - | - |
| Microcrystalline cellulose (Sterculia urens gum and dextrin surface treatment, microcrystalline cellulose:Sterculia urens gum:dextrin = 80:10:10 (weight ratio)) | - | 5 | - |
| Microcrystalline cellulose | - | - | 3.75 |
| Xanthan gum | - | - | 0.6 |
| Dextrin | - | - | 1 |
| Sorbitol | 15 | 10 | 15 |
| Propylene glycol | 5 | - | 5 |
| Butylene glycol | - | 3 | - |
| Cetylpyridinium chloride | 0.1 | 0.1 | 0.1 |
| Sodium saccharin | 0.2 | 0.2 | 0.2 |
| Dicalcium phosphate dihydrate | 35 | - | 35 |
| Silica | 4 | 5 | 4 |
| Calcium carbonate | - | 30 | - |
| Cocamidepropyl betaine | 1 | - | 1 |
| Alkylglycoside | - | 2.5 | - |
| Flavor | 1 | 1 | 1 |
| Purified water | balance | balance | balance |
| Total | 100 | 100 | 100 |
| Shape retention (after 20 seconds) | 9 | 12 | 9 |
| Stringiness (mm) | 14.3 | 15.2 | 22.1 |
| Viscosity (Pa·s) | 22 | - 35 | 33 |

As is apparent from the results shown in Table 4, when microcrystalline cellulose surface-treated with a water-soluble substance is blended with the oral hygiene composition, viscosity becomes lower and stringiness is suppressed even in the case of having the same shape retention as that of the oral hygiene composition blended with each material alone.

In the same manner as in the dispersibility test 1, dispersibility of cetylpyridinium chloride as an active ingredient in the oral hygiene compositions of Reference Examples 3 and 4 as well as Comparative Example 3 was evaluated. The results are shown in Table 5.

**Table 5**

| Shaking time | Elution rate (%) | | |
|---|---|---|---|
| | Example 3 (Reference) | Example 4 (Reference) | Comparative Example 3 |
| 5 seconds | 2 | 2 | 1 |
| 15 seconds | 3 | 8 | 1 |
| 30 seconds | 6 | 44 | 2 |
| 60 seconds | 33 | 60 | 21 |
| 120 seconds | 90 | 70 | 55 |
| 180 seconds | 98 | 80 | 61 |

As is apparent from the results shown in Table 5, the oral hygiene compositions of Reference Examples 3 and 4 showed enhanced dispersibility from 5 seconds after shaking and most of them were completely dispersed after 3 minutes. In contrast, the oral hygiene composition of Comparative Example 3 showed low dispersibility. After 3 minutes when the oral hygiene composition of Example 3 was almost completely dispersed, only 61% of the oral hygiene composition was dispersed.

### Dispersibility Test 2

In order to compare the difference in dispersibility in a short shaking time, dispersibility of each active ingredient of the oral hygiene compositions of Example 2 and Comparative Example 2 was examined under the same conditions except that a rotating speed of a shaker was changed to 320 times/min. The results are shown in Table 6.

**Table 6**

| | Pharmacologically active ingredient | Cetylpyridinium chloride | Triclosan | Vitamin E (Tocopheryl acetate) | Sodium fluoride | Tranexamic acid | Dipotassium glycyrrhizate | Average (%) |
|---|---|---|---|---|---|---|---|---|
| Sample | Time (seconds) | Elution rate (%) | Elution rate (%) | Elution rate (%) | Elution rate (%) | Elution rate (%) | Elution rate (%) | |
| Example 2 | 15 | 45 | 41 | 42 | 57 | 49 | 45 | 47 |
| | 30 | 52 | 49 | 50 | 62 | 55 | 52 | 54 |
| | 60 | 63 | 61 | 62 | 72 | 66 | 63 | 65 |
| | 120 | 74 | 70 | 71 | 85 | 77 | 73 | 75 |
| | 180 | 80 | 75 | 77 | 89 | 83 | 78 | 80 |
| Comparative Example 2 | 15 | 7 | 7 | 7 | 12 | 8 | 8 | 8 |
| | 30 | 10 | 12 | 12 | 16 | 13 | 13 | 13 |
| | 60 | 28 | 29 | 29 | 33 | 31 | 30 | 30 |
| | 120 | 41 | 44 | 45 | 45 | 46 | 44 | 45 |
| | 180 | 54 | 56 | 56 | 55 | 58 | 57 | 56 |

As is apparent from the results shown in Table 6, since the oral hygiene composition of Example 2 showed enhanced dispersibility immediately after shaking and enabled various blended ingredients to immediately disperse throughout the oral cavity when it was applied to the oral cavity, it can be used with an excellent feeling for use and can sufficiently exert the effects of various ingredients.

As a result of total consideration of the results of Experimental Examples 1 and 2, the oral hygiene compositions blended with microcrystalline cellulose surface-treated with a water-soluble substance of the present invention have higher shape retention and lower stringiness than those in the case of the oral hygiene compositions blended with the same raw material ingredients in the same ratio and amount. All of these oral hygiene compositions of the Examples can be extruded from a pump container and are suited for use as an oral hygiene composition for a pump.

### Example 5

**Toothpaste**

| Ingredients | Amount (% by weight) |
|---|---|
| Microcrystalline cellulose (sodium carboxymethyl cellulose surface treatment, microcrystalline cellulose:sodium carboxymethyl cellulose = 85:15 (weight | 3.0 |
| ratio)) | |
| Hydroxyethyl cellulose | 1.0 |
| Lauryl glucoside | 3.0 |
| Decyl glucoside | 1.0 |
| Calcium hydrogenphosphate | 30.0 |
| Cetylpyridinium chloride | 0.1 |
| Pyridoxine hydrochloride | 0.1 |
| Sodium monofluorophosphate | 0.72 |
| Flavor | 1.0 |
| Sodium saccharin | 0.1 |
| Titanium dioxide | 0.3 |
| Propylene glycol | 3.0 |
| Glycerin | 20.0 |
| Purified water | balance |
| Total | 100 |

### Example 6 (Reference)

**Toothpaste**

| Ingredients | Amount (% by weight) |
|---|---|
| Microcrystalline cellulose (xanthan gum surface treatment, microcrystalline cellulose:xanthan gum = 75:5) | 3.0 |
| Decyl glucoside | 2.0 |
| Cocamidopropyl betaine | 1.0 |
| Silicic anhydride | 30.0 |
| Sodium carboxymethyl cellulose | 2.0 |
| Vitamin E (tocopheryl acetate) | 0.05 |
| Sodium fluoride | 0.2 |
| Methylparaben | 0.2 |
| Flavor | 1.0 |
| Sodium saccharin | 0.1 |
| Titanium dioxide | 0.3 |
| Sorbitol | 30.0 |
| Purified water | balance |
| Total | 100 |

### Example 7 (Reference)

**Toothpaste**

| Ingredients | Amount (% by weight) |
|---|---|
| Microcrystalline cellulose (dextrin surface treatment, microcrystalline cellulose:dextrin = 80:10) | 2.0 |
| Sucrose ester of lauric acid | 2.0 |
| Calcium pyrophosphate | 35.0 |
| Gellan gum | 0.5 |
| Sodium chloride | 15.0 |
| Tranexamic acid | 0.1 |
| Flavor | 1.0 |
| Sodium saccharin | 0.1 |
| Titanium dioxide | 0.3 |
| PEG-8 | 5.0 |
| Purified water | balance |
| | |
| Total | 100 |

### Example 8 (Reference)

**Toothpaste**

| Ingredients | Amount (% by weight) |
|---|---|
| Microcrystalline cellulose (Sterculia urens gum surface treatment, microcrystalline cellulose:Sterculia urens gum = 80:10) | 2.0 |
| Decaglycerin laurate ester | 2.0 |
| Calcium carbonate | 25.0 |
| Hydroxypropylmethylcellulose | 1.0 |
| Isopropylmethyl phenol | 0.05 |
| PEG-60 hydrogenated castor oil | 1.0 |
| Sodium polyoxyethylene (2E.O.) alkyl (C₁₂₋₁₄) sulfosuccinate | 1.0 |
| Stevia extract | 0.1 |
| Flavor | 1.0 |
| Sodium saccharin | 0.1 |
| Titanium dioxide | 0.3 |
| Glycerin | 10.0 |
| Xylitol | 10.0 |
| Purified water | balance |
| Total | 100 |

### Example 9 (Reference)

### Toothpaste

| Ingredients | Amount (% by weight) |
|---|---|
| Microcrystalline cellulose (sodium carboxymethyl cellulose surface treatment, microcrystalline cellulose:sodium carboxymethyl cellulose = 80:20) | 1.5 |
| Algin | 1.0 |
| Sodium polyacrylate | 0.5 |
| β-glycyrrhetinic acid | 0.01 |
| Allantoin | 0.02 |
| Angelica acutiloba root extract | 0.01 |
| Aluminum hydroxide | 15.0 |
| Hyroxyapatite | 5.0 |
| Tetrasodium pyrophosphate | 5.0 |
| Zeolite | 2.0 |
| Maltitol | 3.0 |
| Dextranase | 0.1 |
| Sodium benzoate | 0.1 |
| Alkylglycoside | 2.0 |
| Hydroxyethylimidazolinium betain | 0.02 |
| Malic acid | 0.05 |
| Potassium hydroxide | 0.02 |
| PCA ethyl cocoyl arginate | 0.01 |
| PEG-60 hydrogenated castor oil | 1.0 |
| Flavor | 1.0 |
| Purified water | balance |
| Total | 100 |

### Example 10 (Reference)

### Toothpaste

| Ingredients | Amount (% by weight) |
|---|---|
| Microcrystalline cellulose (sodium carboxymethyl cellulose surface treatment, microcrystalline cellulose:sodium carboxymethyl cellulose = 85:15) | 2.0 |
| Carrageenan | 1.5 |
| Sodium lauroyl sarcosinate | 1.3 |
| Polyglycerol esters of fatty acid | 1.0 |
| Calcium carbonate | 20.0 |
| Sodium polyphosphate | 3.0 |
| Benzethonium chloride | 0.01 |
| Polyethylene glycol | 1.0 |
| 6-aminocaproic acid | 0.05 |
| Zinc oxide | 0.5 |
| Flavor | 1.0 |
| Isomalt | 1.0 |
| Xylitol | 2.0 |
| Ultramarines | 0.01 |
| Erythritol | 5.0 |
| Sodium silicate | 0.1 |
| Purified water | balance |
| Total | 100 |

Example 11 (Reference)

### Creamy dentifrice

| Ingredients | Amount (% by weight) |
|---|---|
| Microcrystalline cellulose (sodium carboxymethyl cellulose surface treatment, microcrystalline cellulose:sodium carboxymethyl cellulose = 89:11) | 1.5 |
| Cetylpyridinium chloride | 0.05 |
| Dipotassium glycyrrhizate | 0.1 |
| Triclosan | 0.1 |
| Silica | 10.0 |
| Xanthan gum | 0.5 |
| Glycerin | 40.0 |
| Propylene glycol | 3.0 |
| Sodium lauryl sulfate | 1.0 |
| Titanium dioxide | 0.5 |
| Sodium saccharin | 0.1 |
| Tocopheryl nicotinate | 0.2 |
| PEG-60 hydrogenated castor oil | 2.0 |
| Flavor | 1.0 |
| Purified water | balance |
| Total | 100 |

### Example 12 (Reference)

**Creamy dentifrice**

| Ingredients | Amount (% by weight) |
|---|---|
| Microcrystalline cellulose (sodium carboxymethyl cellulose surface treatment, microcrystalline cellulose:sodium carboxymethyl cellulose = 90:10) | 1.5 |
| Cetylpyridinium chloride | 0.05 |
| Abrasive precipitated silica | 8.0 |
| Thickening silica | 2.0 |
| Xanthan gum | 0.5 |
| Sodium fluoride | 0.2 |
| Sorbitol | 55.0 |
| Propylene glycol | 3.0 |
| Sodium lauryl sulfate | 1.5 |
| Titanium dioxide | 0.3 |
| Sodium saccharin | 0.1 |
| Tocopheryl nicotinate | 0.2 |
| PEG-60 hydrogenated castor oil | 1.0 |
| Flavor | 1.0 |
| Purified water | balance |
| Total | 100 |

### Example 13 (Reference)

### Creamy dentifrice

| Ingredients | Amount (% by weight) |
|---|---|
| Microcrystalline cellulose (sodium carboxymethyl cellulose surface treatment, microcrystalline cellulose:sodium carboxymethyl cellulose:xanthan gum:dextrin = 73:5:3:19) | 2.5 |
| Cetylpyridinium chloride | 0.05 |
| Calcium hydrogenphosphate | 20.0 |
| Thickening silica | 4.0 |
| Xanthan gum | 0.7 |
| Sodium monofluorophosphate | 0.7 |
| Sorbitol | 30.0 |
| Butylene glycol | 3.0 |
| Alkylglycoside | 3.0 |
| Titanium dioxide | 0.2 |
| Sodium saccharin | 0.1 |
| Angelica acutiloba root extract | 0.1 |
| Flavor | 1.0 |
| Purified water | balance |
| Total | 100 |

### Example 14 (Reference)

### Gel-like dentifrice

| Ingredients | Amount (% by weight) |
|---|---|
| Microcrystalline cellulose (xanthan gum surface treatment, microcrystalline cellulose:xanthan gum = 75:5) | 5.0 |
| Myristic acid amidopropylbetain | 0.5 |
| Tetraglycerin laurate ester | 1.0 |
| Tocopheryl acetate | 0.1 |
| Glycerin | 30.0 |
| PEG-8 | 4.0 |
| Butylene glycol | 2.0 |
| Flavor | 1.0 |
| Sodium saccharin | 0.1 |
| Disodium citrate | 0.12 |
| Sodium citrate | 0.01 |
| Purified water | balance |
| Total | 100 |

### Example 15

### Gel-like dentifrice

| Ingredients | Amount (% by weight) |
|---|---|
| Microcrystalline cellulose (sodium carboxymethyl cellulose surface treatment, microcrystalline cellulose: sodium carboxymethyl cellulose = 89:11) | 4.0 |
| Chlorhexidine dihydrochloride | 0.2 |
| Decyl glucoside | 1.0 |
| Sodium fluoride | 0.2 |
| Glycerin | 40.0 |
| PEG-8 | 5.0 |
| Butylene glycol | 2.0 |
| Propylene glycol | 8.0 |
| Flavor | 1.0 |
| Sodium saccharin | 0.1 |
| Disodium phosphate | 0.12 |
| Sodium phosphate | 0.01 |
| Purified water | balance |
| Total | 100 |

The present invention can be utilized as an oral hygiene composition such as a medicated dentifrice having the effect of preventing or treating diseases by enabling various blended ingredients, particularly pharmacologically active ingredients, to act effectively on the affected portion in the oral cavity.

## Claims

1. An oral hygiene composition to be used by ejecting onto a toothbrush from a pump container, the composition comprising surface-treated microcrystalline cellulose having a coating of water-soluble polymer which is sodium carboxymethyl cellulose, contained at a weight ratio of microcrystalline cellulose to said sodium carboxymethyl cellulose in the range from 4:1 to 15:1;
in which the amount of the surface-treated microcrystalline cellulose blended in the oral hygiene composition is within a range from 3 to 7% by weight based on the total amount of the composition; and
the composition further contains a pharmacologically active ingredient which is one or more selected from the group consisting of cetylpyridinium chloride, benzethonium chloride, distearyldimonium chloride, stearalkonium chloride, steartrimonium chloride, steartrimonium chloride, laurylpyridinium chloride, chlorhexidine dihydrochloride, chlorhexidine acetate, chlorhexidine digluconate, alexidine hydrochloride, alexidine acetate, alexidine gluconate, triclosan, O-cymen-5-ol, enzymes, zinc compounds, vitamins and derivatives thereof, amino acids, types of collagen, 6-aminocaproic acid, allantoin and derivatives thereof, dihydrocholesterol, glycyrrhizates, glycyrrhetinic acid, glycerophosphates and chlorophyll.

2. An oral hygiene composition according to claim 1 wherein the pharmacologically active ingredient is one or more selected from the group consisting of cetylpyridinium chloride, triclosan, vitamin E and derivatives thereof, tranexamic acid and glycyrrhizate.

3. An oral hygiene composition according to claim 1 or 2 which further contains an abrasive.

4. An oral hygiene composition according to claim 3 in which the abrasive is selected from abrasive silica, dicalcium phosphate dihydrate, anhydrous dicalcium phosphate dihydrate, calcium carbonate, calcium pyrophosphate, aluminum hydroxide, alumina and hydroxyapatite.

5. An oral hygiene composition according to claim 3 or 4 in which the amount of abrasive is within a range from 4 to 40% by weight based on the total amount of the oral hygiene composition.

6. An oral hygiene composition according to any one of the preceding claims which further contains a surfactant.

7. An oral hygiene composition according to any one of the preceding claims wherein the viscosity of the composition at 30°C is from 5 to 20 Pa·s.

8. An oral hygiene composition according to any one of the preceding claims which is a dentifrice.

9. An oral hygiene composition according to claim 8 which is a toothpaste having a viscosity at 30°C from 5 to 20 Pa·s and which is contained in a pump container.

## Patentansprüche

1. Mundhygienezusammensetzung, die durch das Ausdrücken aus einem Pumpbehälter auf eine Zahnbürste zu verwenden ist, wobei die Zusammensetzung oberflächenbehandelte mikrokristalline Cellulose mit einem Überzug aus wasserlöslichem Polymer umfasst, wobei es sich um Natriumcarboxymethylcellulose handelt, das in einem Gewichtsverhältnis zwischen mikrokristalliner Cellulose und Natriumcarboxymethylcellulose im Bereich von 4:1 bis 15:1 enthalten ist,
wobei die Menge an oberflächenbehandelter mikrokristalliner Cellulose, die der Mundhygienezusammensetzung beigemischt ist, im Bereich von 3 bis 7 Gew.-%, bezogen auf die Gesamtmenge der Zusammensetzung liegt, und
die Zusammensetzung des Weiteren eine pharmakologisch aktiven Inhaltsstoff enthält, wobei es sich um einen oder mehrere ausgewählt aus der aus Cetylpyridiniumchlorid, Benzethoniumchlorid, Distearyldimoniumchlorid, Stearalkoniumchlorid, Steartrimoniumchlorid, Steartrimoniumchlorid, Laurylpyridiniumchlorid, Chlorhexidindihydrochlorid, Chlorhexidinacetat, Chlorhexidindiglukonat, Alexidinhydrochlorid, Alexidinacetat, Alexidinglukonat, Triclosan, O-Cymen-5-ol, Enzymen, Zinkverbindungen, Vitaminen und Derivaten davon, Aminosäuren, Kollagentypen, 6-Aminocapronsäure, Allantoin und Derivaten davon, Dihydrocholesterin, Glycyrrhizaten, Glycyrrhetinsäure, Glycerophosphaten und Chlorophyll bestehenden Gruppe handelt.

2. Mundhygienezusammensetzung nach Anspruch 1, wobei es sich bei dem pharmakologisch aktive Inhaltsstoff um einen oder mehrere ausgewählt aus der aus Cetylpyridiniumchlorid, Triclosan, Vitamin E und Derivaten davon, Tranexamsäure und Glycyrrhizat bestehenden Gruppe handelt.

3. Mundhygienezusammensetzung nach Anspruch 1 oder 2, die des Weiteren ein Schleifmittel enthält.

4. Mundhygienezusammensetzung nach Anspruch 3, wobei das Schleifmittel ausgewählt ist aus abschleifender Kieselerde, Dicalciumphosphatdihydrat, wasserfreiem Dicalciumphosphatdihydrat, Calciumcarbonat, Calciumpyrophosphat, Aluminiumhydroxid, Aluminiumoxid und Hydroxyapatit.

5. Mundhygienezusammensetzung nach Anspruch 3 oder 4, worin die Menge an Schleifmittel in einem Bereich von 4 bis 40 Gew.-%, bezogen auf die Gesamtmenge der Mundhygienezusammensetzung, liegt.

6. Mundhygienezusammensetzung nach einem der vorangegangenen Ansprüche, die des Weiteren ein Tensid enthält.

7. Mundhygienezusammensetzung nach einem der vorangegangenen Ansprüche, wobei die Viskosität der Zusammensetzung bei 30 °C 5 bis 20 Pa·s beträgt.

8. Mundhygienezusammensetzung nach einem der vorangegangenen Ansprüche, wobei es sich um ein Zahnputzmittel handelt.

9. Mundhygienezusammensetzung nach Anspruch 8, wobei es sich um eine Zahncreme mit einer Viskosität von 5 bis 20 Pa·s bei 30 °C handelt, die in einem Pumpbehälter enthalten ist.

## Revendications

1. Composition pour l'hygiène orale à utiliser en éjectant sur une brosse à dent à partir d'un récipient à pompe, la composition comprenant une cellulose microcristalline traitée en surface présentant un revêtement de polymère soluble dans l'eau qui est la carboxyméthylcellulose de sodium, contenue à un rapport massique de cellulose microcristalline à ladite carboxyméthylcellulose de sodium dans l'intervalle de 4:1 à 15:1 ;
dans laquelle la quantité de la cellulose microcristalline traitée en surface combinée dans la composition pour l'hygiène orale se trouve dans un intervalle de 3 à 7 % en masse rapporté à la quantité totale de la composition ; et
la composition contient de plus un ingrédient pharmacologiquement actif qui un ou plusieurs choisis dans le groupe constitué de chlorure de cétylpyridinium, de chlorure de benzéthonium, de chlorure de distéaryldimonium, de chlorure de stéaralkonium, de chlorure de stéartrimonium, de chlorure de stéartrimonium, de chlorure de laurylpyridinium, de dihydrochlorure de chlorhexidine, d'acétate de chlorhexidine, de digluconate de chlorhexidine, d'hydrochlorure d'alexidine, d'acétate d'alexidine, de gluconate d'alexidine, de triclosan, d'O-cymén-5-ol, d'enzymes, de composés du zinc, de vitamines et de dérivés de celles-ci, d'aminoacides, de types de collagène, d'acide 6-aminocaproïque, d'allantoïne et de dérivés de celle-ci, de dihydrocholestérol, de glycyrrhizates, d'acide glycyrrhétinique, de glycérophosphates et de chlorophylle.

2. Composition pour l'hygiène orale selon la revendication 1, dans laquelle l'ingrédient pharmacologiquement actif est un ou plusieurs choisis dans le groupe constitué du chlorure de cétylpyridinium, du triclosan, de la vitamine E et de dérivés de celle-ci, de l'acide tranexamique et du glycyrrhizate.

3. Composition pour l'hygiène orale selon la revendication 1 ou 2, laquelle contient de plus un abrasif.

4. Composition pour l'hygiène orale selon la revendication 3, dans laquelle l'abrasif est choisi parmi la silice abrasive, le phosphate de dicalcium dihydraté, le phosphate de dicalcium dihydraté anhydre, le carbonate de calcium, le pyrophosphate de calcium, l'hydroxyde d'aluminium, l'alumine et l'hydroxyapatite.

5. Composition pour l'hygiène orale selon la revendication 3 ou 4, dans laquelle la quantité d'abrasif se trouve dans un intervalle de 4 à 40 % en masse rapporté à la quantité totale de la composition pour l'hygiène orale.

6. Composition pour l'hygiène orale selon l'une quelconque des revendications précédentes qui contient de plus un tensioactif.

7. Composition pour l'hygiène orale selon l'une quelconque des revendications précédentes dans laquelle la viscosité de la composition à 30°C est de 5 à 20 Pa.s.

8. Composition pour l'hygiène orale selon l'une quelconque des revendications précédentes, laquelle est un dentifrice.

9. Composition pour l'hygiène orale selon la revendication 8, laquelle est une pâte dentifrice ayant une viscosité à 30°C de 5 à 20 Pa.s et laquelle est contenue dans un récipient à pompe.
